(19) Europäisches Patentamt
European Patent Office
Office européen des brevets

(11) Veröffentlichungsnummer: **0 103 273**
**B1**

(12) # EUROPÄISCHE PATENTSCHRIFT

(45) Veröffentlichungstag der Patentschrift: **27.12.85**

(51) Int. Cl.[4]: **C 07 C 43/178**, C 07 C 41/28, C 07 C 43/13, C 07 C 69/02, C 07 C 143/68

(21) Anmeldenummer: **83108855.4**

(22) Anmeldetag: **08.09.83**

(54) **Verfahren zur Herstellung von Glyzerinethern und ihre Verwendung.**

(30) Priorität: **14.09.82 DE 3234040**

(43) Veröffentlichungstag der Anmeldung:
**21.03.84 Patentblatt 84/12**

(45) Bekanntmachung des Hinweises auf die Patenterteilung:
**27.12.85 Patentblatt 85/52**

(84) Benannte Vertragsstaaten:
**AT BE CH DE FR GB IT LI NL SE**

(56) Entgegenhaltungen:
**JOURNAL OF THE AMERICAN CHEMICAL SOCIETY, Band 84, Nr. 12, 29. Juni 1962, Seiten 2371-2377, American Chemical Society, USA E.L. ELIEL et al.: "Reduction with metal hydrides. XII. Reduction of acetals and ketals with lithium aluminum hydride-aluminum chloride"**
**TETRAHEDRON LETTERS, Nr. 39, September 1979, Seiten 3551-3554, Pergamon Press, Oxford, GB. Oxford, GB. A. LIPTAK et al.: "Hydrogenolysis of the dioxolan type EXO- and ENDO- benzylidene derivatives of carbohydrates with the LiA1H4-A1C13 reagent"**
**DIE PHARMAZIE, 33. Jahrgang, Heft 4, April 1978, Seiten 181-183, DD. P. NUHN et al.: "Synthese einiger 1.2-Di-O-hexadecylglycerophosphor säurepropylester"**

(73) Patentinhaber: **BEHRINGWERKE Aktiengesellschaft, Postfach 1140, D-3550 Marburg 1 (DE)**

(72) Erfinder: **Kolar, Cenek, Dr., Lindenweg 14, D-3550 Marburg/Lahn (DE)**

(74) Vertreter: **Meyer-Dulheuer, Karl-Hermann, Dr. et al, HOECHST Aktiengesellschaft Zentrale Patentabteilung Postfach 80 03 20, D-6230 Frankfurt/Main 80 (DE)**

**EP 0 103 273 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft ein Verfahren zur Herstellung einer R- oder S-stereoisomeren oder racemischen Verbindung der allgemeinen Formel I

$$R^1—OCH_2—CH(OR^2)—CH_2O—R^3 \quad (I)$$

worin $R^1$ eine Benzylgruppe ist, $R^2$ und $R^3$ verschieden sind und eines von ihnen ein Wasserstoffatom und das andere eine $C_{1-22}$-Alkylgruppe bedeuten, dadurch gekennzeichnet, daß man eine 1,2- oder 1,3-O-Benzyliden-Verbindung der allgemeinen Formel I, worin $R^1$ und $R^2$ oder $R^1$ und $R^3$ zusammen eine Benzylidengruppe sind und jeweils $R^3$ oder $R^2$ eine $C_{1-22}$-Alkylgruppe ist, in Gegenwart einer Lewissäure zu einer R- oder S-stereoisomeren oder racemischen Benzylether-Verbindung der allgemeinen Formel I reduziert, wobei bei einer racemischen Verbindung $R^1$ und $R^3$ untereinander austauschbar sind.

Die Erfindung betrifft weiterhin die Verwendung dieser Verbindung in einem Verfahren zur Herstellung einer R- oder S-stereoisomeren oder racemischen Verbindung der Formel II

$$HOCH_2—CH(OR^2)—CH_2O—R^3 \quad (II)$$

worin entweder $R^2$ oder $R^3$ eine $C_{1-22}$-Alkylgruppe ist und $R^3$ und $R^2$ verschieden sind und eine $C_{1-22}$-Alkyl-, $C_{1-16}$-Alkanoyl- oder Methansulfonylgruppe bedeuten.

Diese Verbindungen der Formel II dienen als wertvolle Zwischenprodukte zur Herstellung von optisch aktiven oder racemischen gemischtkettigen etherischen Phospholipid-Derivaten, bevorzugt Kephalin- oder Lecithin-Analoga.

Etherische Analoga der Phospholipide besitzen wertvolle pharmakologische Eigenschaften. Aufgrund ihrer physikalisch-chemischen Eigenschaften (oberflächenaktive Wirkung besitzen sie einen großen Einfluß auf die Permeabilitätsverhältnisse in Zellmembranen. Insbesondere die gemischtkettigen etherischen Phospholipid-Analoga, das heißt Phospholipide mit einer langen und einer kurzen etherisch an ein Glycerinsegment gebundenen Alkylkette, beeinflussen in vitro und in vivo die Eigenschaften der Zellmenbranen.

Diese Phospholipid-Analoga wurden als Immunmodulatoren und zur Inhibition von Tumorwachstum und Metastasenbildung in vivo angewandt (Seventh International Symposium on Immunopathology, Ed. Miescher, P.A., Schwabe & Co., Publishers, Basel, Seiten 411—424 (1976).

In der Deutschen Offenlegungsschrift 26 19 686 ist eine Synthese von gemischkettigen etherischen Phospholipid-Analoga beschrieben. Nach diesem Verfahren können nur racemische 1,2-Di-O-alkylglycerin-Derivate hergestellt werden. Außerdem ist dieses Verfahren, worin 2-O-Alkylglycerin verethert wird, nicht genügend selektiv. Es bilden sich als Nebenprodukte 1,2,3-Tri-O-alkylglycerine. Wegen der Nebenprodukte ist die Reinigung der Zwischen- und Endprodukte aufwendig und kostspielig.

In der Publikation von G. Hirth und R. Barner (Helvetica Chimica Acta, Vol. 65, Fasc. 3, 1982 Nr. 100) wurde die Herstellung von 3-O-Benzyl-1-O-octadecyl-sn-glycerin beschrieben. Diese Verbindung wurde entweder durch direkte Veretherung von 3-O-Benzyl-sn-glycerin, die nicht genügend selektiv verläuft, oder durch mehrstufige Synthese aus einem tosylierten Zwischenprodukt hergestellt.

Aufgabe der vorliegenden Erfindung war es deshalb, einen neuen Weg zur selektiven Synthese von 3-(oder 1)-O-Alkyl-2-O-alkyl(oder acyl-)-glycerin-Derivaten bereitzustellen, der die aufgezeigten Nachteile vermeidet und es ermöglicht, Glycerinphosphatide stellungsspezifisch, optisch aktiv oder racemisch und mit hoher Selektivität auf einfache und wirtschaftliche Weise herzustellen.

Bevorzugt werden nach dem erfindungsgemäßen Verfahren R- oder S-stereoisomere oder racemische Verbindungen der Formel I hergestellt, worin $R^1$ eine Benzylgruppe ist und $R^2$ und $R^3$ verschieden sind und das eine von ihnen ein Wasserstoffatom und das andere $-(CH_2)_n-CH_3$ mit n = 0,11 oder 17 bedeuten, die als Zwischenprodukte zur Herstellung von R- oder S-stereoisomeren oder racemischen 1-O-Alkyl-2-O-alkyl (oder alkanoyl)-glycerin-Verbindungen der Formel II verwendet werden, worin $R^2$ und $R^3$ verschieden sind, $R^2$ eine Acetyl-, Methansulfonylgruppe oder $-(CH_2)_n-CH_3$ mit n = 0, 11 oder 17 und $R^3$ $-(CH_2)_n-CH_3$ mit n = 0, 11 oder 17 bedeuten.

Diese Verbindungen werden zur Herstellung von Phospholipid-Derivaten der Formel III verwendet

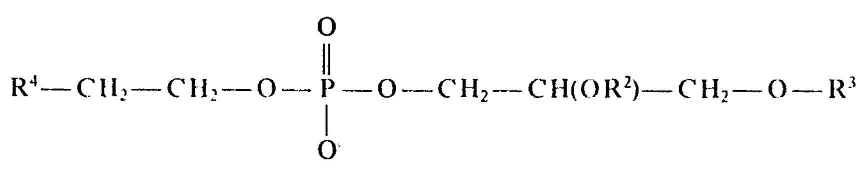

(III)

worin $R^2$ und $R^3$ die oben angegebene Bedeutung besitzen und $R^4$ $-N^+H_3$ oder $N^+(CH_3)_3$ ist.

Gegenstand der vorliegenden Erfindung ist deshalb ein Verfahren zur Herstellung einer Verbindung der Formel I, worin $R^1$ eine Benzylgruppe ist, $R^2$ und $R^3$ verschieden sind und das eine von ihnen ein Wasserstoffatom und das andere eine $C_{1-22}$-Alkylgruppe bedeuten, dadurch gekennzeichnet, daß man R- oder S-stereoisomeres oder racemisches 1-O-Alkyl-2,3-O-benzyliden-glycerin oder 2-O-Alkyl-1,3-O-benzyliden-glycerin, worin Alkyl die für $R^2$ oder $R^3$ allgemein angegebene Bedeutung hat, mit $LiAlH_4$, $B_2H_4$ oder $AlCl_3$ in Diethylether, Dioxan, THF, Dichlormethan oder deren Mischungen bei einer Temperatur von −20°C bis 100°C, bevorzugt 0°C bis 25°C zur Benzylether-Verbindung der Formel I umsetzt.

Gegenstand der Erfindung ist weiterhin die Verwendung einer Verbindung der Formel I zur Herstellung einer Verbindung der Formel II, worin entweder $R^2$ oder $R^3$ eine $C_{1-22}$-Alkylgruppe ist und $R^3$ und $R^2$ verschieden sind und eine $C_{1-22}$-Alkyl-, $C_{1-16}$-Alkanoyl- oder Methansulfonylgruppe bedeuten, indem eine Verbindung der Formel I in an sich bekannter Weise mit einem Alkylhalogenid oder Sulfonsäurealkylester in Gegenwart einer Base verethert und anschließend die verbleibende Benzylgruppe hydrogenolytisch abgespalten oder in an sich bekannter Weise mit einem Alkanoyl-anhydrid oder - halogenid oder Methansulfonylchlorid in Gegenwart einer Base verestert und anschließend die verbleibende Benzylgruppe hydrogenolytisch abgespalten wird.

Durch das erfindungsgemäße Verfahren werden die bekannten Nachteile der Synthese gemischkettiger Glycerinderivate, wie beispielsweise 1-O-Alkyl-2-O-alkyl (oder alkanoyl)-glycerinen, insbesondere die nicht selektive Veretherung von 2-O-Alkyl-glycerinen, vermieden. Es hat sich gezeigt, daß die nach dem erfindungsgemäßen Verfahren hergestellten Verbindungen besonders geeignete Ausgangsmaterialien zur Herstellung von gemischtkettigen Glycerinderivaten sind. Die Schutzgruppen können selektiv abgelöst werden, wobei jeder einzelne Verfahrensschritt unter Bedingungen durchgeführt werden kann, von denen die übrigen Substituenten nicht beeinflußt werden.

Das erfindungsgemäße Verfahren stellt eine sehr selektive, einfache und wirtschaftliche Methode zur Herstellung von Glycerinderivaten dar, die vor allem für die stellungsspezifische Darstellung von optisch aktiven oder racemischen Glycerinderivaten mit verschiedenen Resten von Bedeutung ist. Die nachfolgenden Beispiele erläutern die Erfindung, ohne sie darauf zu beschränken.

## Beispiel 1

### 1,2-O-Benzyliden-3-O-octadecyl-sn-glycerin (Verbindung 1)

10 g (29 mMol) 3-O-Octadecyl-sn-glycerin wurden in 50 ml destilliertem Benzaldehyd gelöst. Nach der Zugabe von 2,5 g Zink(II)chlorid wurde die Mischung 10 Stunden bei Raumtemperatur und dann 4 Stunden bei 40°C gerührt. Nach Abdestillieren von Benzaldehyd im Hochvakuum wurde der Rückstand in Chloroform aufgenommen, und die anorganischen Bestandteile wurden mit Wasser ausgewaschen. Die Chloroform-Phase wurde über Natriumsulfat getrocknet und im Vakuum eingeengt. Der resultierende Sirup wurde chromatographisch gereinigt (Petrolether/Ether 2 : 1).
Ausbeute: 10,2 g.
IR (Chloroform, $cm^{-1}$): 3060, 3030, 2930, 2850, 1470, 1455, 1110.

| | | |
|---|---|---|
| berechnet: | C 77,77 | H 11,11 |
| gefunden: | C 77,82 | H 11,14 |

1,2-O-Benzyliden-3-O-dodecyl-sn-glycerin (Verbindung 2).
Verbindung 2 wurde entsprechend synthetisiert und IR- und NMR-spektroskopisch charakterisiert.

| | | |
|---|---|---|
| berechnet: | C 75,86 | H 10,34 |
| gefunden: | C 75,91 | H 10,39 |

## Beispiel 2

### 1,2-O-Benzyliden-3-O-octadecyl-glycerin (Verbindung 3)

10 g (55,4 mMol) 1,2-O-Benzyliden-glycerin wurden in 100 ml trockenem Toluol gelöst. Nach der Zugabe von 7,5 g Kalium tert.-butylat und 19,4 g (55,4 mMol) Methansulfonsäure-octadecylester wurde die Suspension auf 70°C erwärmt und 40 Minuten unter Stickstoffatmosphäre gerührt. Nach Abkühlen wurde die Suspension mit Toluol versetzt, und die Salze wurden mit Wasser ausgewaschen. Nach Einengen der organischen Phase wurde das Rohprodukt chromatographisch gereinigt (Chloroform/Essigester 10 : 1).
Ausbeute: 22,3 g.
Die racemische Verbindung 3 ist nach der dünnschicht-chromatographischen und IR-spektroskopischen Untersuchung identisch mit Verbindung 1.
Analog wurden folgende racemische Verbindungen synthetisiert:

1,2-O-Benzyliden-3-O-dodecyl-glycerin (Verbindung 4).
Verbindung 4 ist nach IR-Untersuchung identisch mit Verbindung 2.
1,2-O-Benzyliden-3-O-methyl-glycerin (Verbindung 5).
$^{13}$C.NMR (100 MHz, $CDCl_3$) delta: C-2 75,2 (75,9) ppm; PhCH 104,2 (104,1); $CH_3$ 59,1 (59,2).

| | | |
|---|---|---|
| berechnet: | C 68,04 | H 7,21 |
| gefunden: | C 68,21 | H 7,23 |

Beispiel 3

1-O-Benzyl-3-O-octadecyl-sn-glycerin (Verbindung 6)

3,5 g (8,1 mMol) Verbindung 1 oder 3 wurden in einer Mischung aus 40 ml Ether und 40 ml Dichlormethan gelöst. Nach der Zugabe von 1,5 g $LiAlH_4$ wurden bei Raumtemperatur 4,3 g $AlCl_3$ gelöst in 40 ml Ether tropfenweise zugegeben. Nach 15 Minuten Reaktionszeit zeigte das Dünnschichtchromatogramm (Chloroform/Essigester 9 : 1) eine vollständige Umsetzung an. Das überschüssige Hydrid wurde mit Wasser ausgewaschen, die organische Phase über Natriumsulfat getrocknet und im Vakuum zum Sirup eingeengt.
Ausbeute: 3 g. $(\alpha)_D^{20} = -1{,}62°$ (c = 5 in $CHCl_3$).
$^{13}$C-NMR (100 MHz, $CDCl_3$) delta: Phenyl 137,9, 128,2, 127,9 ppm; Glycerin C-1 72,0; C-2 69,5; C-3 73,3.

| | | |
|---|---|---|
| berechnet: | C 77,42 | H 11,52 |
| gefunden: | C 77,50 | H 11,53 |

Entsprechend wurden folgende Verbindungen synthetisiert:
1-O-Benzyl-3-O-methyl-sn-glycerin (Verbindung).
$^{13}$C-NMR (100 MHz, $CDCl_3$) delta: C-1 71,4 ppm; C-2 69,4; C-3 73,4; $CH_3$ 59,1; $PhCM_2$ 73,8.
1-O-Benzyl-3-O-dodecyl-sn-glycerin (Verbindung 8).
IR ($CHCl_3$, $cm^{-1}$): 3440, 3060, 3020, 2910, 2840, 1495, 1465, 1110.

Beispiel 4

2-O-Methyl-3-O-octadecyl-sn-glycerin (Verbindung 9)

10 g (23,0 mMol) Verbindung 6 wurden in 80 ml trockenem Dioxan gelöst. Unter Feuchtigkeitsausschluß wurden unter Rühren 3,4 g (29 mMol) Kalium tert.-butylat und 3,7 g (25,3 mMol) Methyljodid gelöst in 20 ml trockenem Dioxan zugegeben. Nach 15 Minuten Rühren bei Raumtemperatur (DC: Petrolether/Essigester 5 : 1) wurde die Reaktionsmischung i. Vak. eingeengt. Der in 100 ml aufgenommene Rückstand wurde zweimal mit 5%iger (5 g/100 ml) wäßriger NaCl-Lösung gewaschen. Die organische Phase wurde mit Natriumsulfat getrocknet und i. Vak. eingeengt. Das einheitliche Rohprodukt wurde ohne weitere Reinigungsschritte für die folgende Hydrierung eingesetzt. Das Produkt wurde IR- und NMR-spektroskopisch charakterisiert.
$(\alpha)_{589}^{20} = 0°$ (c = 1 in $CHCl_3$).

10 g Rohprodukt wurden in 100 ml trockenem Eisessig gelöst und in Gegenwart von 7 g Palladiumkohle (10 Gew.-%) hydriert. Dann wurde abfiltriert, eingeengt, Toluol zugegeben und abdestilliert. Der resultierende Sirup kristallisierte in Ether/Petrolether aus.
Ausbeute: 7,3 g.
Schmp. = 45°C; $(\alpha)_D^{20} = +8{,}41°$ (c = 1 in $CHCl_3$).
$^{13}$C-NMR (100 MHz, $CDCl_3$) delta: C-1 63,2 ppm; C-2 81,2; C-3 71,5; $CH_3$ 58,5.

| | | |
|---|---|---|
| berechnet: | C 73,74 | H 12,84 |
| gefunden: | C 73,78 | H 12,91 |

Analog wurden folgende Verbindungen synthetisiert:
2-O-Dodecyl-3-O-octadecyl-sn-glycerin (Verbindung 10).
$(\alpha)_{589}^{20} = -9{,}2°$ (c = 1 in $CHCl_3$).

| | | |
|---|---|---|
| berechnet: | C 77,28 | H 13,36 |
| gefunden: | C 76,57 | H 13,30 |

2-O-Dodecyl-3-O-octadecyl-sn-glycerin (Verbindung 11).
$(\alpha)_{589}^{20} = -7{,}9°$ (c = 1 in $CHCl_3$).

berechnet: C 77,28 H 13,36
gefunden: C 77,21 H 13,40

2-O-Methyl-3-O-dodecyl-glycerin (Verbindung 12).

berechnet: C 70,02 H 12,49
gefunden: C 70,12 H 12,53

2-O-Octadecyl-3-O-methyl-glycerin (Verbindung 13).

berechnet: C 73,68 H 12,93
gefunden: C 73,77 H 12,96

2-O-Dodecyl-3-O-methyl-glycerin (Verbindung 14).

berechnet: C 70,02 H 12,49
gefunden: C 70,10 H 12,56

## Beispiel 5

### 1-O-Benzyl-2-O-methyl-glycerin (Verbindung 15)

10,7 g (55,5 mMol) 1,3-O-Benzyliden-2-O-methyl-glycerin wurden in einer Mischung aus 100 ml Ether und 100 ml Dichlormethan gelöst. Nach der Zugabe von 10 g $LiAlH_4$ wurden bei Raumtemperatur 30 g $AlCl_3$, gelöst in 100 ml Ether, tropfenweise zugegeben. Nach 30 Minuten Reaktionszeit wurde das überschüssige $LiAlH_4$ mit Essigester zersetzt. Die organische Phase wurde ausgewaschen, über Natriumsulfat getrocknet und i. Vak. eingeengt.
Ausbeute: 9,5 g.
$^{13}C$-NMR (100 MHz, $CDCl_3$) delta: C-1 69,6 ppm; C-2 80,2; C-3 62,3; $PhCH_2$ 73,5; $CH_3$ 57,7.

berechnet: C 68,39 H 6,73
gefunden: C 68,12 H 6,68

## Beispiel 6

### 1-O-Benzyl-2-O-methyl-3-O-octadecyl-glycerin (Verbindung 16)

10,8 g (55,4 mMol) Verbindung 15 wurden in 100 ml trockenem Toluol gelöst. Nach der Zugabe von 7,5 g Kalium tert.-butylat und 19,4 g (55,4 mMol) Methansulfonsäureoctadecylester wurde die Suspension auf 70°C erwärmt und 40 Minuten unter Stickstoffatmosphäre gerührt. Nach Abkühlen wurde die Suspension mit Toluol versetzt und mit Wasser ausgewaschen. Die organische Phase wurde eingeengt und der resultierende Sirup säulenchromatographisch gereinigt (Petrolether/Essigester 10 : 1).
Ausbeute: 22 g.

berechnet: C 77,67 H 11,60
gefunden: C 77,81 H 11,63

Die Verbindung 16 wurde, wie im Beispiel 4 beschrieben, in Gegenwart von Palladiumkohle hydriert und aufgearbeitet, wobei die racemische Verbindung 9 entstand.

### 1-O-Benzyl-2-O-methyl-3-O-dodecyl-glycerin (Verbindung 17 a)

Die Verbindung 17 a wurde wie im Beispiel 6 beschrieben hergestellt und weiter zu 3-O-Dodecyl-2-O-methyl-glycerin (Verbindung 17 b) umgesetzt.
Verbindung 17 a.
IR ($CHCl_3$, $cm^{-1}$): 3080, 3050, 3020, 2910, 2840, 1490, 1465, 1450, 1110.
Verbindung 17 b entspricht dem IR-Spektrum nach der Verbindung 12.

Beispiel 7

2-O-Acetyl-1-O-benzyl-3-O-octadecyl-sn-glycerin (Verbindung 18)

7 g (16,1 mMol) 1-O-Benzyl-3-O-octadecyl-sn-glycerin wurden mit Acetanhydrid in Gegenwart von Pyridin acetyliert und in üblicher Weise aufgearbeitet.
Ausbeute: 7,5 g.
$(\alpha)_D^{20} = -1,75°$ (c = 5 in $CHCl_3$).
IR (Chloroform, $cm^{-1}$): 1745 (Carbonyl), 1600 (Benzyl), 1120 (Ether).

| | | |
|---|---|---|
| berechnet: | C 75,58 | H 10,99 |
| gefunden: | C 75,60 | H 11,01 |

Beispiel 8

2-O-Acetyl-3-O-octadecyl-sn-glycerin (Verbindung 19)

7 g (14,7 mMol) Verbindung 18 wurden in 100 ml Methanol/Essigester 1 : 1 gelöst und in Gegenwart von 2 g Palladiumkohle (10 Gew.-%) hydriert. Das nach Filtrieren und Eindampfen i. Vak. erhaltene Produkt wurde aus Diisopropylether/Petrolether kristallisiert.
Ausbeute: 5,5 g.
Schmp. = 47°C, $(\alpha)_D^{20} = +9,65°$ (c = 1 in $CHCl_3$).

Beispiel 9

2-O-Methansulfonyl-3-O-octadecyl-sn-glycerin (Verbindung 20)

7 g (16,1 mMol) 1-O-Benzyl-3-O-octadecyl-sn-glycerin wurden in 25 ml Pyridin und 25 ml trockenem Chloroform gelöst. Bei −10°C wurden 1,76 g (17 mMol) Methansulfonsäurechlorid, gelöst in 25 ml Chloroform, unter Rühren und Feuchtigkeitsausschluß zugetropft. Danach wurde die Temperatur auf +10°C erhöht und die Reaktionsmischung noch 3 Stunden gerührt (DC: Chloroform/Essigester 9 : 1). Die Reaktionsmischung wurde mit Chloroform versetzt und dreimal mit Eiswasser gewaschen. Nach Einengen der organischen Phase i. Vak. wurde die 2-O-Mesyl-Verbindung ohne weitere Reinigung weiter umgesetzt.

8,2 g mesyliertes Rohprodukt wurden wie im Beispiel 17 beschrieben mit Palladiumkohle hydriert und aufgearbeitet.
Ausbeute: 5,6 g.
Schmp. = 51°C.
IR (KBr, $cm^{-1}$); 3570, 2950, 1360, 1180, 1120.

| | | | |
|---|---|---|---|
| berechnet: | C 62,51 | H 10,96 | S 7,58 |
| gefunden: | C 62,70 | H 10,92 | S 7,5 |

**Patentansprüche**

1. Verfahren zur Herstellung einer Verbindung der Formel I

$$R^1—OCH_2—CH(OR^2)—CH_2O—R^3 \qquad (I)$$

worin $R^1$ eine Benzylgruppe ist, $R^2$ und $R^3$ verschieden sind und eines von ihnen ein Wasserstoffatom und das andere eine $C_{1-22}$-Alkylgruppe bedeuten, dadurch gekennzeichnet, daß man eine R- oder S-stereoisomeres oder racemisches 1-O-Alkyl-2,3-O-benzyliden-glycerin oder 2-O-Alkyl-1,3-O-benzyliden-glycerin, worin Alkyl die für $R^2$ oder $R^3$ allgemein angegebene Bedeutung hat, mit $LiAlH_4$, $B_2H_4$ oder $AlCl_3$ in Diethylether, Dioxan, THF, Dichlormethan oder einer Mischung aus diesen bei einer Temperatur von −20°C bis 100°C zur Benzylether-Verbindung der Formel I umsetzt.

2. Verwendung einer nach Anspruch 1 hergestellten Verbindung zur Herstellung einer Verbindung der Formel II

$$HOCH_2—CH(OR^2)—CH_2O—R^3 \qquad (II)$$

worin entweder $R^2$ oder $R^3$ eine $C_{1-22}$-Alkylgruppe ist und $R^3$ und $R^2$ verschieden sind und eine $C_{1-22}$-Alkyl-, $C_{1-16}$-Alkanoyl- oder Methansulfonylgruppe bedeuten, indem eine Verbindung der Formel I in an

sich bekannter Weise mit einem Alkylhalogenid oder Sulfonsäurealkylester in Gegenwart einer Base verethert und anschließend die verbleibende Benzylgruppe hydrogenolytisch abgespalten oder in an sich bekannter Weise mit einem Alkanoyl-anhydrid oder -halogenid oder Methansulfonylchlorid in Gegenwart einer Base verestert und anschließend die verbleibende Benzylgruppe hydrogenolytisch abgespalten wird.

## Claims

1. Process for preparing a compound of formula I

$$R^1—OCH_2—CH(OR^2)—CH_2O—R^3 \qquad (I)$$

wherein $R^1$ is benzyl, $R^2$ and $R^3$ are different and one is hydrogen and the other is $C_{1-22}$-alkyl, which comprises allowing to react R or S stereoisomeric or racemic 1-O-alkyl-2,3-O-benzyliden-glycerol or 2-O-alkyl-1,3-O-benzyliden-glycerol, wherein alkyl has the meaning given above for $R^2$ or $R^3$, with $LiAlH_4$, $B_2H_4$ or $AlCl_3$ in diethylether, dioxan, THF, dichloromethan or a mixture of those at a temperature form −20°C to 100°C to form a benzylether of formula I.

2. Use of a compound prepared according to claim 1 for preparing a compound of formula II

$$HOCH_2—CH(OR^2)—CH_2O—R^3 \qquad (II)$$

wherein either $R^2$ or $R^3$ is $C_{1-22}$-alkyl and $R^3$ and $R^2$ are different and denote $C_{1-22}$-alkyl, $C_{1-16}$-alkanoyl or methansulfonyl, by reacting by a method known to the art a compound of formula I and a haloalkane or a sulfonic acid alkylester in presence of a base to form an ether and subsequently cleaving hydrogenolytically the remaining benzylether or by reacting that compound of formula I by a method known to the art with an alkanoyl anhydride or halogenide or methane sulfonyl chloride in presence of a base to form an ester ans subsequently cleaving hydrogenolytically the remaining benzylether.

## Revendications

1. Procédé pour la préparation d'un composé de formule I

$$R^1—OCH_2—CH(OR^2)—CH_2O—R^3 \qquad (I)$$

dans laquelle $R^1$ est un groupe benzyle, $R^2$ et $R^3$ sont différents et l'un d'eux représente un atome d'hydrogène et l'autre un groupe alkyle en $C_{1-22}$, caractérisé par le fait que l'on transforme en éther benzylique de formule I le 1-O-alkyl-2,3-O-benzylidène-glycérol ou le 2-O-alkyl-1,3-O-benzylidène-glycérol racémiques ou leurs stéréoisomères R ou S, dans lesquels le radical alkyle a la signification générale donnée pour $R^2$ ou $R^3$, avec $LiAlH_4$, $B_2H_4$ ou $AlCl_3$ dans du diéthyléther, du dioxanne, du THF, du dichlorométhane ou dans un mélange de ceux-ci, à une température de −20°C à 100°C.

2. Utilisation d'un composé préparé selon la revendication 1 pour la préparation d'un composé de formule II

$$HOCH_2—CH(OR^2)—CH_2O—R^3 \qquad (II)$$

dans laquelle ou $R^2$ ou $R^3$ est un groupe alkyle en $C_{1-22}$ et $R^3$ et $R^2$ sont différents et représentent chacun un groupe alkyle en $C_{1-22}$, alcanoyle en $C_{1-16}$ ou méthanesulfonyle, en éthérifiant un composé de formule I d'une façon connue en soi avec un halogénure d'alkyle ou un sulfonate d'alkyle en présence d'une base et en séparant ensuite par hydrogénolyse le groupe benzyle restant, ou en l'estérifiant d'une façon connue en soi avec un anhydride ou un halogénure d'alcanoyle ou du chlorure de méthanesulfonyle en présence d'une base et en séparant ensuite par hydrogénolyse le groupe benzyle restant.